# EUROPEAN PATENT APPLICATION

(11) **EP 2 233 104 A1**
(43) Date of publication of application: **29.09.2010**
(21) Application number: 10167644.3
(22) Date of filing: 09.06.2006
(51) Int. Cl.: A61C 3/00, A61C 1/02, A61B 17/16, A61C 3/02, B23B 47/04, B25F 5/00, A61C 1/14

(54) **Adjustable tool drive arrangement**

(30) Priority: 10.06.2005 US 689052 P; 01.11.2005 US 262959
(62) Divisional of application: 06761047.7
(71) Applicant: TTi Turner Technology Instruments Inc., Ottawa, ON K1Y 4S7 (CA)
(72) Inventor: Bailey, Kevin John, Ottawa Ontario K1Z 5M4 (CA); Millson, Andrew Douglas, Ottawa Ontario K2B 6A8 (CA); Turner, Derek M. J., Ottawa Ontario K1N 8J7 (CA); Castonguay, Jean, Hudson Québec J0P 1H0 (CA)
(74) Representative: Eisenführ, Speiser & Partner

(57) **Abstract**

The invention relates to an improved dental tool drive arrangement for a handpiece with a drive head, the tool drive arrangement permitting length adjustment of the tool in the drive head by concentrically supporting the tool in the drive head at any position from a fully inserted position to a maximum retracted position. The tool drive arrangement preferably includes a tool and a rotatable tool supporting element for concentrically supporting the tool from the fully inserted to the maximum retracted position, the tool preferably including a maximum retraction indicator for indicating to a user when the tool has been retracted to the maximum retraction position. This provides a significant advantage over the prior art by allowing a user to adjust the exposed length of a rotatable tool, preferably a dental bur, without exceeding safe operating limits. The invention also relates to an improved drive spindle which allows depth adjustment of a tool in a dental handpiece while maintaining efficient torque transfer and concentricity during high speed rotation.

## Description

### CROSS-REFERENCE TO RELATED APPLICATION

This application claims priority from United States Application SN 60/689,052, entitled Dental Burr And Drive Spindle, filed June 10, 2005, and from United States Application SN 11/262,959, entitled Adjustable Tool Drive Arrangement, filed November 1, 2005, which applications are included herein by reference in their entirety.

### FIELD OF THE INVENTION

The present invention relates generally to handpieces for rotating tools. More particularly, the present invention relates to an improved drive arrangement for a rotatable tool, including a drive spindle and the tool.

### BACKGROUND OF THE INVENTION

Numerous handpieces for rotating tools exist. Turbine driven handpieces are widely used in dental offices and medical labs around the world. Most handpieces include a handle and drive head for supporting the rotating tool. A connector, often a swivel connector, connects the handpiece to various air, water, light and power supply conduits, generally combined in a so-called umbilical cord. The drive head houses a tool drive arrangement, generally composed of a tool retaining mount or chuck, and a motor or turbine, rotatably mounted in the head for driving the chuck. The chuck releasably holds the tool, such as a dental bur, for rotation about an axis of rotation.

In known handpieces, the tool is releasably held by the chuck against axial movement in the drive arrangement. Screw lock or pushbutton lock arrangements are provided for the manual locking and releasing of the tool in and from the chuck. The known drive arrangements are not designed to allow for length adjustment of the tool, which means the tool, once fully inserted in the drive arrangement will always protrude the same length from the drive head. However, as a dental procedure progresses, a dentist may need to use dental tools of different length. This creates the need for repeated tool changes, which is time consuming and cost intensive, since a collection of different length tools must be purchased.

In an attempt to find a time and cost efficient solution, dentists often try to adjust the protruding length of the bur by somewhat retracting the bur from the drive head until the desired length is reached. However, this adjustment is made without knowledge whether the bur will remain properly engaged within the drive mechanism and safely secured within the drive head. This is a dangerous practice, since prior art handpieces are not designed to hold the bur in any position other than fully inserted into the drivehead. The tool when retracted may remain within the drive head in the prior art handpieces due to the retaining force of the friction arms normally included in the chuck. However, concentrical support of the tool within the drive head and reliable torque transmission from the drive to the tool are not ensured.

Conventional handpiece designs provide for concentrical support of the tool in the fully inserted condition. Support is provided at a rear, inserted end of the tool and at an intermediate location of the tool corresponding to the area of the bottom bearing in the drivehead. However, upon even a minor retraction of the tool from the fully inserted position, the tool is disengaged from the concentrical support at the rear end of the tool. The tool must then be maintained in axial alignment with the rotating drive by way of the friction arms of the chuck. However, those friction arms are somewhat flexible by design and generally do not provide sufficient force to maintain the rear end of the tool concentrically aligned in the drive when lateral forces are applied to the working end of the tool during use. Therefore, operation of a conventional handpiece at a tool insertion depth other than fully inserted can result in loss of concentricity, vibration of the bur during rotation, excessive wear, damage to the drive assembly, permanent deformation of the tool securing mechanism and drive spindle components, inefficient torque transfer, increased bur slippage (both rotational and axial), and most dangerously, accidental disengagement of the bur from the handpiece during use.

Therefore, a need exists for a dental tool and handpiece design allowing for tool depth adjustment without a loss of concentricity.

Prior art chucks of dental handpieces are almost exclusively designed to hold the dental bur by way of friction fit only. Examples of such constructions are found in US Patents US 3,869,796, US 4,595,363, US 5,275,558, and US 5,549,474. Only low torque transmission is possible between the chuck and the bur in such constructions, higher torque leading to slippage of the bur. At the high rotational speeds achieved by modem dental handpieces, bur slippage, in both the axial and rotational directions, can become a problem. Rapid deceleration of the bur can also lead to rotational slippage, for example, when the drive continues to rotate while the bur is locked or snagged. Friction between the drive assembly and the dental bur during rotation leads to significant wear of both elements over time. This friction can also produce significant heat, as can friction generated in push-button lock handpieces when the user maintains pressure on the push-button during operation. Friction heat can cause permanent damage to the drive spindle components, especially the flexible friction arms of the chuck, which are normally made of heat tempered material. The damage can lead to rotational slippage and even axial slippage of the tool, possibly resulting in an accidental release of the tool from the handpiece. Accidental release of a dental bur during high speed rotation can pose a threat to both the patient and the dentist. Continued wear of the bur and drive assembly during operation necessitates routine maintenance and repair of expensive handpiece components.

Thus, a drive spindle design is desired which not only allows for adjustment of the exposed tool lengths but preferably also prevents rotational slippage of the tool at all possible tool retraction positions to avoid frictional wear and resulting heat damage to the drive spindle.

### SUMMARY OF THE INVENTION

It is an object of the present invention to obviate or mitigate at least one disadvantage of prior art handpiece designs.

In a first aspect, the invention provides a tool drive arrangement for a handpiece with a drive head, the tool drive arrangement permitting length adjustment of the tool in the drive head by concentrically supporting the tool in the drive head at any position from a fully inserted position to a maximum retracted position.

In a preferred embodiment, the tool drive arrangement includes a tool and a rotatable tool supporting element for concentrically supporting the tool from the fully inserted to the maximum retracted position, the tool including a maximum retraction indicator for indicating to a user when the tool has been retracted to the maximum retraction position. This provides a significant advantage over the prior art by allowing a user to adjust the exposed length of a rotatable tool, preferably a dental bur, without exceeding safe operating limits.

In a preferred embodiment of the tool, the tool includes a tool body having an axis of rotation, the tool body being divided into a driven portion with a driven end for insertion into the tool supporting element, and a working portion for projection from the drive head during use. The tool further includes a maximum retraction indicator on the driven portion for indicating to a user when the tool is retracted from the fully inserted position to the maximum retraction position.

In another preferred embodiment, the tool supporting element is a drive spindle for concentrically supporting the tool at different insertion depths from a maximum insertion depth at the fully inserted position to a minimum insertion depth at the maximum retraction position. The drive spindle includes a drive torque receiving portion, a tool supporting portion with a tool passage for receiving the driven portion of the tool and a tool retaining member for releasably retaining the driven portion in the tool passage. The tool supporting portion includes a first tool seat for supporting the drive end of the tool and a second tool seat for supporting the driven portion at a location intermediate the driven end and the working portion of the tool. The first seat has a sufficient axial length for concentrically supporting the driven end when the tool is retracted from the fully inserted position to a retracted position wherein the retaining member still engages the driven portion.

In one aspect, the maximum retraction indicator is a visible indicia located on the driven portion, intermediate the driven end and the working portion, to be hidden from view when the tool is inserted at a depth between the maximum and minimum insertion depths and visible to a user when the tool is retracted from the drive head to the maximum retraction position or further. Preferably, the maximum retraction indicator is selected from the group of at least one dot, line, colored line, etched line, a line having a surface roughness different from the remainder of the driven portion, a change in diameter of the tool and a groove. The line or groove can be continuous or broken, such as a line of dots. The line or groove can extend in circumferential or longitudinal direction of the tool or at any angular orientation therebetween. The maximum retraction depth can be indicated by an end or an edge of the line or groove. The maximum retraction depth can also be indicated by a change in the overall appearance of the line or groove, such as a change in color, a change in size, a change in any other characteristic, or any combination thereof.

In another aspect, the maximum retraction indicator is a mechanical indicia located on the driven portion for engagement by a portion of the tool supporting element when the maximum retraction depth is reached. Preferably, this mechanical engagement provides a tactile indication, possibly even an auditory indication (click), to the user that the maximum retraction depth is reached. In a preferred embodiment, the mechanical indicia is a stop on the driven portion of the tool for mechanical interaction with the tool retaining member of the tool supporting element when the tool is retracted to the maximum retraction depth. Preferably, the tool supporting element includes a tool retaining member for frictionally retaining the tool and the tool further includes a contact surface on the driven portion for engagement by the tool retaining member at insertion depths from the maximum insertion depth to at least the minimum insertion depth. The stop is preferably a stop shoulder on the contact surface of the tool for axial engagement by the tool engaging member when the tool is retracted from the maximum insertion depth to the maximum retraction depth.

In one variant, the contact surface is a detent on the driven portion and the stop is an axial end shoulder of the detent. In a particularly preferred embodiment, frictional engagement of an elongated detent by the tool retaining member allows the tool to be positioned in the handpiece at any insertion depth between the minimum insertion depth (or maximum extraction depth) and the maximum insertion depth. In another variant, the tool comprises two or more detents on the driven portion, each having a stop shoulder for axial engagement with the tool engaging member for defining one or more intermediate insertion depths between the minimum tool insertion depth and the maximum retraction depth. In a particularly preferred embodiment, the detent is a groove extending circumferentially about the driven portion of the tool.

Those skilled in the art will appreciate that the tool insertion depth indicator and tool retaining member can be achieved by other means than those described in the preferred embodiments of the invention without deviating from the essence of the invention. It will also be apparent that more than one tool retaining member can be provided in the tool supporting element while preserving the core function.

It is a significant advantage, of an adjustable length tool drive arrangement in accordance with the invention allowing axial adjustment of tool insertion depth in a dental handpiece, that the number of times a dentist must exchange tools for selection of different tool lengths during the course of a dental procedure is reduced. This reduces the time required to perform the procedure and can reduce operating cost, since fewer tools of specific length need to be purchased and maintained. It is another significant advantage that, by providing the preferred maximum retraction indicator, excessive wear and damage due to insufficient insertion of the tool in the handpiece are avoided.

In an alternate embodiment, the minimum tool insertion depth indicator is a combination of a mechanical and a visual indicator.

In a preferred embodiment of the tool supporting element, the drive spindle has a chuck including the tool supporting portion and the tool retaining member. The tool passage is an axial bore in the chuck for receiving the driven portion of the tool up to the maximum insertion depth. The tool retaining member is a resilient tool engaging member for releasably frictionally engaging a contact surface on the driven portion of the tool from an engagement depth, at which depth contact between the tool engaging member and the driven portion is initiated during tool insertion, to the maximum insertion depth. In a preferred embodiment, the tool engaging member is shaped and constructed to axially engage a maximum retraction depth indicator on the tool in the form of a stop shoulder on the contact surface, when the tool is retracted from the maximum insertion depth to a maximum retraction depth located between the maximum insertion depth and the engagement depth. The tool engaging member is shaped and constructed to frictionally engage the driven portion to prevent axial movement of the tool in the spindle during operation of the handpiece.

In a preferred embodiment, the chuck is a generally cylindrical member having the tool receiving axial bore. A portion of the wall surrounding the bore is resiliently deformable and forms the resilient tool engaging member to allow insertion of the driven portion of the tool into the bore. When the tool is inserted, the chuck wall portion forming the tool engaging member radially inwardly engages the driven portion to frictionally retain the tool in the bore. Axial engagement of the tool engaging member with a first stop shoulder on the contact surface of the tool provides a maximum tool retraction indication.

In a particularly preferred embodiment, the resilient wall portion of the chuck forms of a pair of diametrically opposed axially extending retaining arms, at least one of which has a radially inwardly projecting protrusion extending therefrom for frictionally engaging the contact surface of the tool and for axially engaging a mechanical retraction depth indicator on the tool, such as the maximum retraction depth indicator. In a preferred embodiment, the drive spindle of the present invention further comprises a ram for selectively forcing apart the retaining arms to allow insertion and/or removal of the tool. The ram is axially aligned with and adjacent the chuck in the drive spindle, and operatively engages the chuck in a torque transfer arrangement. In a particularly preferred variant, a hollow cylindrical sleeve is provided for supporting the chuck and the ram in this axially aligned configuration. Various tool drive arrangements are contemplated in accordance with the present invention, which can allow for torque transfer from the drive to the sleeve, from the drive to the chuck, from the drive to the ram, or from the drive directly to the rotatable tool.

Other aspects and features of the present invention will become apparent to those ordinarily skilled in the art upon review of the following description of specific embodiments of the invention in conjunction with the accompanying figures.

### BRIEF DESCRIPTION OF THE DRAWINGS

Embodiments of the present invention will now be described, by way of example only, with reference to the attached Figures, wherein:
Fig. 1 is a cross-sectional view of a known dental handpiece suitable for use with a tool drive arrangement in accordance with the present invention;
Fig. 2 illustrates a perspective view of a rotatable tool and drive spindle components of a tool drive arrangement in accordance with a preferred embodiment of the present invention;
Figs. 3A to 3F show perspective views of dental tools including different types of maximum retraction indicators in accordance with various preferred embodiments of the tool aspect of the invention;
Fig. 3G shows an alternative preferred embodiment of the dental tool with two mechanical depth indicators, including the maximum retraction indicator, located on the locking portion of the tool, as well as a visual indicator of maximum retraction;
Fig. 4A is an axial end view of the dental bur shown in Fig. 3D illustrating a torque lock;
Fig. 4B is an axial end view from the driven end of the dental bur of Fig. 3F or 3G illustrating an alternative torque lock to that exemplified in Fig. 4A;
Fig 4C shows a cross-sectional end view through the locking portion of the dental bur of Fig. 3G taken through line A-A;
Fig. 5 illustrates a perspective view of a preferred embodiment of a dental bur type tool of the invention having a mechanical maximum retraction indicator in the form of a single, axially elongated detent for continuous depth adjustment;
Figs. 6A and 6B illustrate perspective front and rear end views of a preferred embodiment of a chuck of the tool supporting element aspect of the present invention;
Figs. 6C and 6D illustrate alternative preferred embodiments of the chuck of the tool supporting element aspect of the present invention, illustrating a double-tab variant (6C) and a single-tab (or asymmetrical tab) variant (6D);
Fig. 7 illustrates an end view from the tool receiving end of the drive spindle of Fig. 2, but shown in assembled condition;
Fig. 8 illustrates an end view from the driven end of the drive spindle of Fig. 7;
Figs. 9A and 9B illustrate axial cross-sections of the tool drive arrangement of Fig. 2;
Fig. 10 illustrates an axial cross-section through the tool and tool drive arrangement combination of Fig. 2, in an assembled condition and with the dental tool of Fig. 3D inserted into the spindle to the maximum insertion depth;
Fig. 11 illustrates the assembled drive arrangement and tool combination shown in Fig. 10, but with the tool retracted to the maximum retraction length;
Fig. 12 illustrates an alternative preferred embodiment of the tool drive arrangement of the invention having a drive spindle and the asymmetrical chuck of Fig. 6D, in combination with the tool of Fig. 3G;
Figs. 13A and 13B illustrate axial cross-sections through the drive arrangement of Fig. 12 in an assembled condition with Fig. 13B rotated 90° about the axis in relation to Fig. 13A.;
Fig. 14 illustrates an axial cross-section through the drive arrangement as shown in Fig. 13A, with a dental tool as shown in Fig. 3G inserted into the spindle; and
Fig. 15 shows a cross-section through the locking socket of the sleeve of Fig. 13B, taken along line C-C, and illustrates a locking portion of the tool of Fig. 3G positioned therein.

### DETAILED DESCRIPTION OF THE PREFERRED EMBODIMENTS

Generally, the present invention provides a tool drive arrangement for a handpiece with a drive head, the tool drive arrangement permitting length adjustment of a tool in the drive head by concentrically supporting the tool in the drive head at any position from a fully inserted position to a maximum retracted position.

In one embodiment, the invention provides a tool drive assembly including the tool drive arrangement, a rotatable tool and a rotatable tool supporting element for concentrically supporting the tool from the fully inserted position to the maximum retracted position. The tool preferably includes a maximum retraction indicator for indicating to a user when the tool has been retracted to the maximum retraction position. This provides a significant advantage over the prior art by allowing a user to adjust the exposed length of a rotatable tool, preferably a dental bur, without exceeding safe operating limits.

More particularly, the rotatable tool drive assembly in accordance with the invention includes a rotatable tool and a tool supporting element for releasably supporting the tool. The tool supporting element is insertable into a drive head for coaxial rotation in the drive head. The tool has a tool body having an axis of rotation and is divided into a driven portion, with a driven end for insertion into the tool supporting element, and a working portion for projecting from the drive head during use. The tool supporting element has a tool passage for coaxially receiving the driven portion of the tool and supporting it at different insertion depths, the tool passage including a first tool seat for concentrically supporting the driven end of the tool and a second tool seat for concentrically supporting the driven portion at a location intermediate the driven end and the working portion. In a preferred embodiment, the first tool seat is axially elongated for concentrically supporting the driven end at any position from a maximum insertion position, wherein the tool is fully inserted into the tool passage, to a maximum retraction position, wherein the tool is retracted from the maximum insertion position.

The invention will now be described in more detail with reference to specific preferred embodiments of the invention directed to an improved tool drive arrangement and tool, wherein the tool is a dental tool such as a bur, and the tool supporting element is a drive spindle, such as a drive spindle for use in a high speed turbine-driven dental handpiece. Although specific reference is made in the following to a dental bur and a drive spindle for a high speed dental handpiece, it will become apparent to those skilled in the art that all structural and functional features of the invention are equally applicable to rotatable dental and medical tools in general and to medical and dental handpieces and other handpieces for supporting high speed rotating tools.

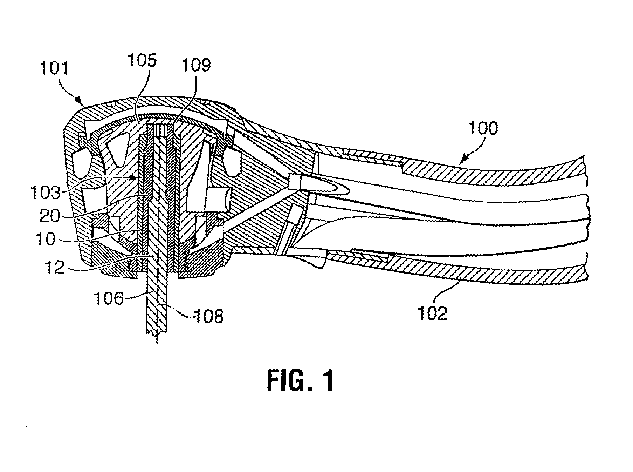

A high speed dental handpiece 100, as shown in Fig. 1 generally includes a handle 102, a tool supporting drive head 101, and a swivel connector (not illustrated) for connecting the handpiece to various air, water, light and power supply conduits, generally combined in a so called umbilical cord (not shown). The drive head 101 includes a torque producing drive 105, typically a motor or turbine rotatably mounted in the drive head, and having a spindle socket 109 for housing a tool supporting element 103, here a drive spindle 10. The tool supporting element 103 typically includes a tool receiving and retaining portion, here a chuck 20, constructed to releasably retain a tool 106, such as a dental bur, for rotation about an axis of rotation 108. The tool supporting element 103 may be retained in the drive head 101 by any means known in the art, for example, by press-fitting the tool supporting element 103 in the spindle socket 109 of the drive head.

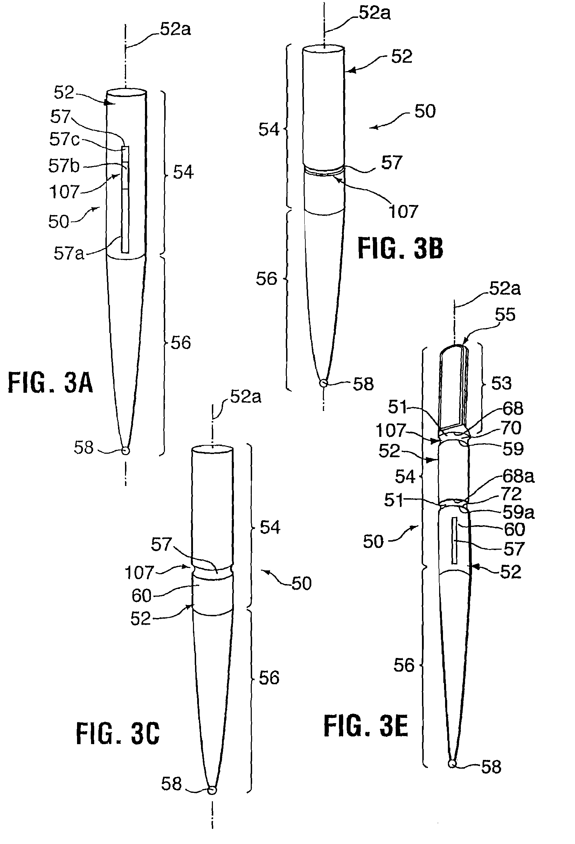

Referring now to Figs. 1 to 3G, a dental tool 106, such as the dental bur 50, typically has an elongated body 52 divided into a generally cylindrical driven portion 54 for insertion into the drive head 101 of a dental handpiece 100 for receiving drive torque from the drive 105 of the handpiece, and a working portion 56 for projecting from the drive head 101 of the handpiece in an operating condition. The working portion has a working end 58 for engagement with a working surface, such as a tooth surface (not illustrated), during a dental procedure. The user, typically a dentist, must purchase a collection of burs varying in shaft length as well as in the structure of the working end 58 of the working portion 56. The dental bur 50 is generally inserted into the spindle 10 in the drive head 101 and is removably supported therein by the chuck 20 for rotation with the spindle 10 about the axis of rotation 108.

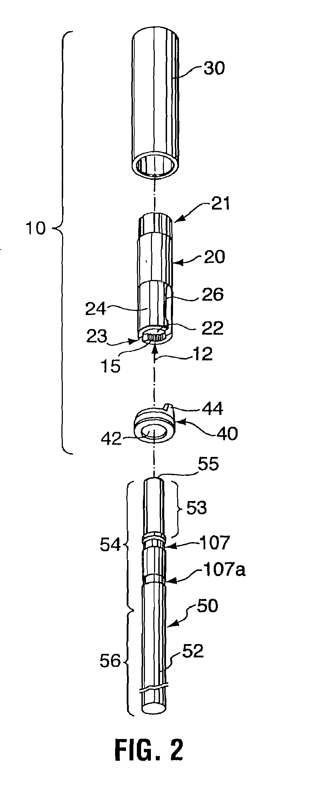

As illustrated in Fig. 2, a preferred embodiment of the tool drive arrangement of the present invention provides an improved drive spindle 10, to be described in more detail below, for use with an improved dental bur 50 in accordance with the tool aspect of the invention.

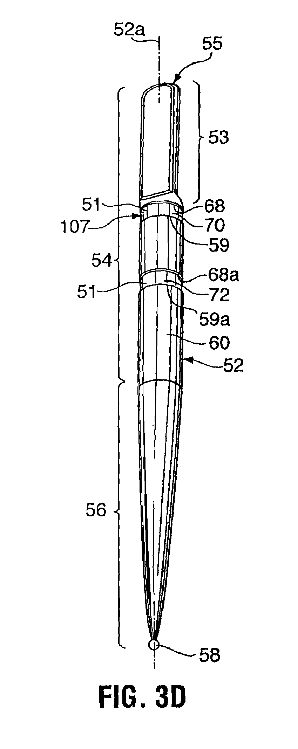

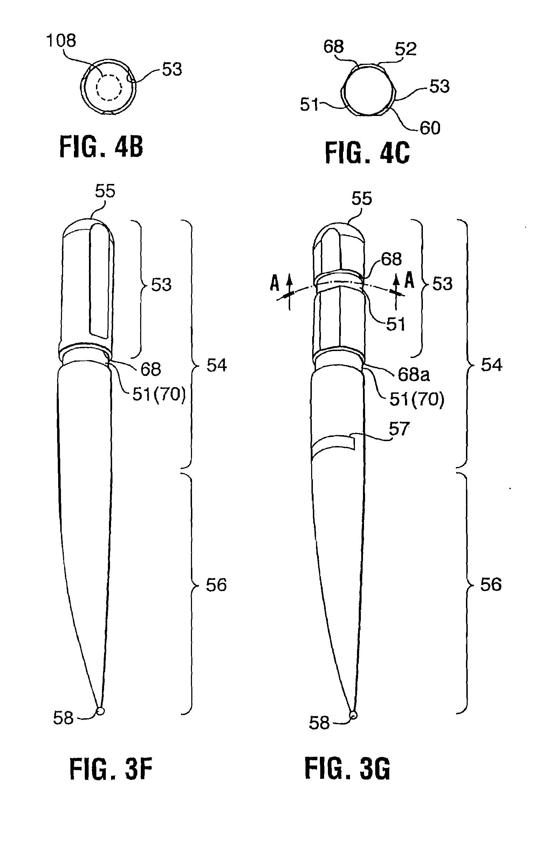

Different preferred embodiments of the tool aspect of the present invention are now described by reference to the various preferred dental bur embodiments shown in Figs. 3A to 3G. The dental bur 50 illustrated in Figs. 3A to 3G includes a body 52 having an axis of rotation 52a, a working portion 56 for projecting from the drive head 101 (see Fig. 1) of a dental handpiece 100 during use, and a driven portion 54 for insertion into the drive head for directly or indirectly receiving drive torque. All illustrated burs include a maximum retraction indicator 107, which can be either a visible indicator 57 as shown in the burs of Figs. 3A to 3C, a mechanical indicator 59 as shown in the bur of Fig. 3D or 3F, or a combination of visible and mechanical indicator as shown in Figs. 3E and 3G.

A visible indicator 57, as shown in the burs of Figs. 3A to 3C, 3E and 3G is preferably provided on or in the surface of the driven portion 54 of the bur 50 such that the indicator is not visible at the maximum tool insertion depth. When the driven portion 54 is retracted from the maximum insertion depth toward the maximum retraction depth, for depth adjustment, the visual indicator 57 becomes apparent to the user, preferably only when the maximum tool retraction position (or minimum tool insertion depth) is reached. A few examples of visual indicia include, but are not limited to, a dot, a line, a colored line, an etched line, a laser mark, a line having a surface roughness different from the remainder of the driven portion, a detent and a groove. The visual indicator 57 can extend completely or partially circumferentially about the driven portion 54 as shown in Figs. 3B and 3G, respectively, axially along the driven portion 54 as shown in Fig. 3A, or at an angular orientation to the axis of rotation 108 (not shown). If the visible indicator 57 extends axially as shown in Fig. 3A, the maximum retraction position can be indicated by the start or end of the indicator, by an edge of the indicator, or by a change in the overall appearance of the indicator, such as a change in color, a change in size, a change in any other characteristic, or any combination thereof. This is shown in Fig. 3A which illustrates a bur 50 with a visual indicator 57 having sections 57a to 57c of different characteristics (preferably colour), whereby the maximum extraction depth is indicated by the transition from section 57b to section 57c becoming visible to the user.

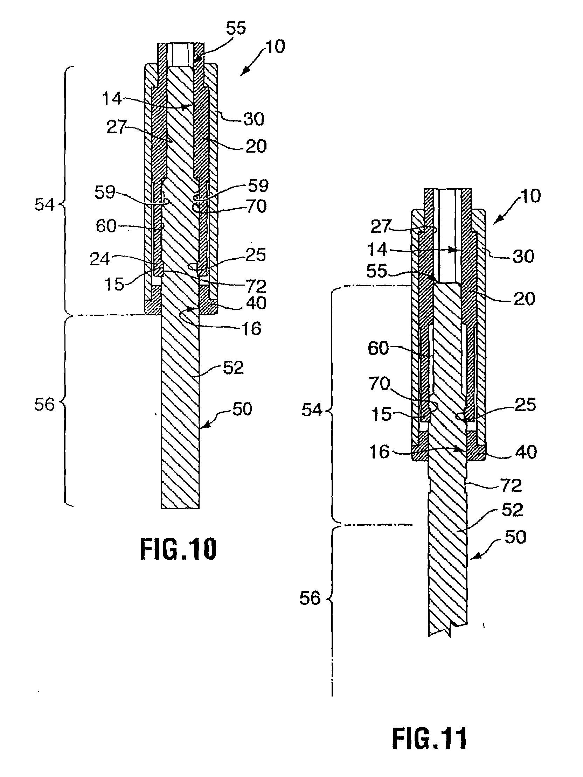

As exemplified in the embodiment of Fig. 3D, the bur 50 has a contact surface 60 on the driven portion 54 for frictional engagement by a tool engaging member 15 of the spindle 10, to be discussed in more detail below in relation to Fig. 2. The tool engaging member 15 engages the contact surface 60 at insertion depths of the tool from an engagement depth, at which contact between the tool engaging member 15 and the driven portion 54 is initiated, to a maximum insertion depth, at which the bur 50 is fully inserted into the handpiece 100. In this embodiment, the mechanical type maximum retraction indicator 59 includes a first stop shoulder 68 on the contact surface 60 for axial engagement with the tool engaging member 15 when the bur 50 is retracted from the maximum insertion depth (Dₘₐₓ) to a maximum retraction depth (Dₘᵢₙ) between the engagement depth and the maximum insertion depth (see Figs. 10 and 11).

The bur of Fig. 3E includes both the visible indicator 57 shown in Fig. 3B and the mechanical indicator 59 shown in Fig. 3D. The bur shown in Fig. 3G includes a visible indicator 57 in the form of a laser mark as well as the mechanical indicator 59 as shown in Figs. 3D to 3F.

In a preferred embodiment as shown in Fig. 2, the mechanical type maximum retraction indicator 59 is a detent 51 located on the driven portion 54, the detent having a first axial stop shoulder 68 for axial engagement by the tool engaging member 15 of the drive spindle 10 for indicating the minimum insertion depth (Dₘᵢₙ) (or maximum retraction depth) of the bur 50 in the drive spindle 10. Dₘᵢₙ is essentially the depth at which the working portion 56 is maximally extended from the handpiece while the driven portion 54 is still concentrically supported in the drive spindle 10 and properly engaged with the drive mechanism in the handpiece for reliable torque transfer. Dₘᵢₙ can be easily determined for various handpiece and spindle designs without undue experimentation. A conservative Dₘᵢₙ can be also be selected which is greater than the depth at which the working portion 56 is maximally extended from the handpiece. The difference between Dₘᵢₙ and Dₘₐₓ provides a length of axial play along which the bur 50 can be safely adjusted in the drive spindle 10.

As illustrated in the preferred embodiment shown in Fig. 2, a second retraction depth indicator 107a can be provided on the driven portion 54 for defining a corresponding second or intermediate insertion depth of the driven portion 54 in the drive spindle 10 between Dₘᵢₙ and Dₘₐₓ, and including Dₘₐₓ. The preferred embodiment illustrated in Fig. 3D shows two annular circumferential detents 51 on the driven portion 54, those being the maximum retraction depth indicator 59 and an intermediate insertion depth indicator 59a, having first and second stop shoulders 68 and 68a respectively for axial engagement with the tool engaging member 15 upon retraction (see Fig. 2) of the driven portion 54 from the maximum insertion depth toward the intermediate insertion depth, Dₘᵢₙ, or the engagement depth. Axial engagement of the first axial stop shoulder 68 by the tool engaging member 15 indicates that Dₘᵢₙ is reached. In the variant shown in Fig. 3D, axial engagement of the second stop shoulder 68a by the tool engaging member 15 occurs when the tool is inserted to essentially Dₘₐₓ (see Fig. 10). The second stop shoulder 68a thus serves to retain the driven portion 54 at Dₘₐₓ during operation of the handpiece, while the first stop shoulder 68 serves to retain the driven portion 54 at Dₘᵢₙ during operation with a maximally extended bur 50 (see Fig. 11).

The bur exemplified in Fig. 3G also includes an intermediate mechanical retraction depth indicator 59a on the driven portion 54. Intermediate retraction depth indicators (mechanical and/or visual) can be provided on the bur to indicate to a user when the maximum insertion depth (Dₘₐₓ) or any desired intermediate insertion depth has been reached. With the mechanical indicator, the user preferably perceives a tactile indication (i.e. a snap) and/or auditory indication (i.e. a click) upon engagement of a mechanical indicator by the tool engaging member 15.

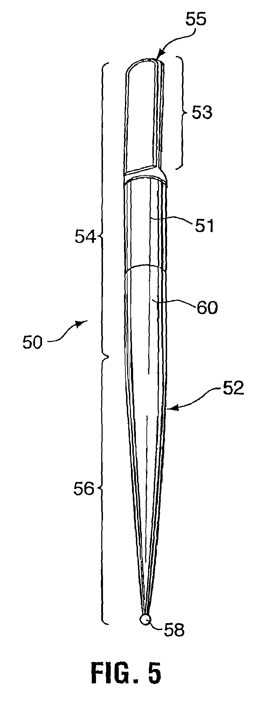

Alternative embodiments of the mechanical retraction depth indicator of the present invention include, but are in no way limited to: (a) a single axially elongated detent 51 on the driven portion 54 as illustrated in Fig. 5, for continuous depth adjustment wherein frictional engagement of the contact surface 60 of the detent 51 by the tool engaging member 15 allows the tool to be securely positioned in the handpiece at any insertion depth between Dₘᵢₙ and Dₘₐₓ during operation of the handpiece, Dₘᵢₙ being indicated by axial engagement of the first stop shoulder 68 by the tool engaging member 15; (b) a plurality of insertion depth indicators 59 as shown in Figs. 3D and 3G, each having a stop shoulder 68 located on the driven portion 54 for defining a plurality of corresponding intermediate insertion depths between Dₘᵢₙ and Dₘₐₓ, Dₘᵢₙ being indicated by the first axial stop shoulder 68 of the maximum retraction depth indicator 59; (c) a contoured annular maximum retraction depth indicator that gradually tapers radially outward axially from the first stop shoulder in the direction of the working portion 56 (not illustrated), wherein frictional engagement of the contact surface of the tapered annular detent by the tool engaging member 15 allows the driven portion 54 to be positioned in the handpiece at any insertion depth between Dₘᵢₙ and Dₘₐₓ, Dₘᵢₙ being indicated by the first axial stop shoulder 68.

Although for ease of manufacture the mechanical indicator 59 described above is preferably in the form of a recessed detent 51 on the contact surface 60 of the driven portion 54, it will be readily understood that the indicator, and especially the stop shoulder 68, could be in the form of an elevation protruding from the surface of the driven portion 54. As will become apparent to persons of skill in the art, other indicator variants can serve as the mechanical indicator to indicate when a desired insertion depth has been reached and as such are considered to be within the scope of the present invention.

In accordance with a preferred embodiment, a detent is any type of recess located on the body 52 of the bur 50, but is preferably an annular, circumferentially extending groove on the driven portion 54. An axially elongated detent or a plurality of axially spaced apart annular detents on the driven portion 54 allow for safe and controlled axial adjustment of the bur 50 in the drive spindle 10 at a range of depths between Dₘₐₓ and a predetermined Dₘᵢₙ, thereby providing for "depth indexing". The provision of safe tool depth adjustment and controlled depth indexing in a dental handpiece satisfies a long felt need in the art.

Operation of the handpiece at tool insertion depths between Dₘᵢₙ and the engagement depth is also possible due to frictional engagement of the driven portion 56 by the tool engaging member 15 but is not preferred due to the disadvantages associated with bur overextension.

The terms "maximum retraction indicator", "maximum retraction depth indicator", "minimum insertion depth indicator", "minimum tool insertion depth indicator", and similar terms, are used interchangeably herein. Similarly, the terms "maximum retraction position", "maximum retraction length", "maximum retraction depth", "minimum insertion depth", and similar terms, are used interchangeably herein. In this context, the terms "retracted" or "retraction" indicate that the tool is retracted from the maximum insertion depth, at which depth the tool is fully inserted into the drive spindle, toward the working end of the drive spindle. In contrast, the terms "inserted" or "insertion" refer to insertion of the tool into the working end of the drive spindle toward the driven end of the spindle.

In a preferred embodiment of the present invention, illustrated in Figs. 2 and 7 to 11, the tool supporting element, in this embodiment the spindle 10, is insertable into the spindle socket 109 of the drive head 101 for coaxial rotation in the drive head. The tool 106, here the bur 50, has a tool body 52 with axis of rotation 108, a driven portion 54 with driven end 55 for insertion into the spindle 10, and a working portion 56 for projecting from the drive head during use. As shown in Figs. 10 and 11, the spindle 10 has a tool passage 12 for coaxially receiving the driven portion 54 of the tool at different insertion depths, the tool passage 12 including a first tool seat 14 for concentrically supporting the driven end 55 of the bur 50 and a second tool seat 16 for concentrically supporting the driven portion 54 of the bur 50 at a location intermediate the driven end 55 and the working portion 56. The first tool seat 14 is axially elongated for concentrically supporting the driven end 55 at any position from a maximum insertion position (Dₘₐₓ), wherein the bur 50 is fully inserted into the tool passage 12 (Fig. 10), to a maximum retraction position (Dₘᵢₙ), wherein the bur 50 is retracted from the maximum insertion position (Fig. 11). In a preferred embodiment of the first tool seat 14, the axial length (depth) of the first tool seat is at least equal to 10% of the axial length of the driven portion 54 of the bur 50 used in combination with the spindle 10. To obtain a sufficiently large retraction length, the axial length (depth) of the first tool seat 14 is more preferably at least 15% of the axial length of the driven portion 54, most preferably at least 20%. Practical retraction ranges are achievable when the axial length (depth) of the first tool seat 14 is 15 to 60% of the axial length of the driven portion 54, more preferably 20 to 75%, although other length ratios are also within the confines of the present invention. The axial length of the first tool seat 14 can also be selected independent of the length of the driven portion 54 of the bur 50 used in combination therewith, preferred seat lengths being at least 1.5mm, more preferably at least about 2mm, more preferably about 2-7mm and most preferably about 5mm.

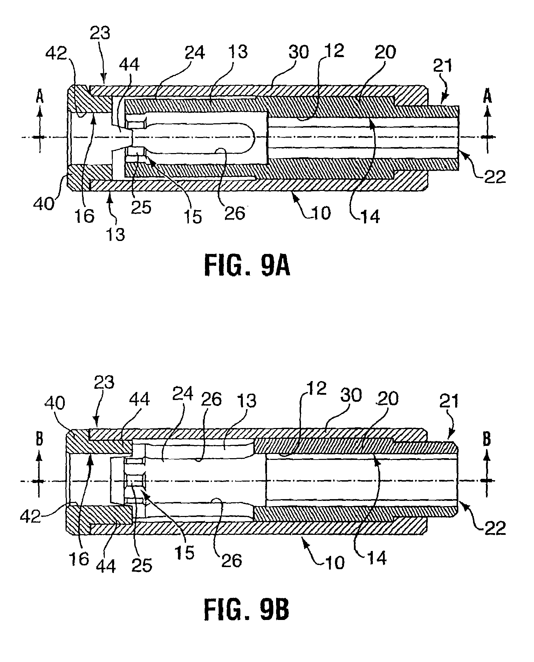

The spindle 10 of the preferred embodiment of the tool supporting element shown in Fig. 2 (as shown in Figs. 9A, 9B, 10, 11) includes a torque receiving element in the form of a generally cylindrical casing sleeve 30 which fits into the spindle socket 109 of the drive head 101 for receiving rotational torque from the drive 105. The casing sleeve 30 houses a tool supporting element, in the form of a chuck 20, for releasably supporting the bur 50, and a ram 40 for selectively releasing the bur 50 from the chuck 20. The chuck 20 includes the tool passage 12 in the form of a tool receiving axial bore 22 for receiving the driven portion 54 of the bur 50 coaxial with the axis of rotation 108. The axial bore preferably extends from a driven chuck end 21 of the chuck 20 to the tool receiving end 23. The chuck 20 further includes a tool retaining member in the form of a resilient tool retaining arm 24. The tool passage 12 includes the first tool seat 14 for supporting the driven end 55 of the bur 50 and the second tool seat 16 for supporting the driven portion 54 at a location intermediate the driven end 55 and the working portion 56. In the spindle 10 embodiment exemplified in Figs. 9A and 9B, the first tool seat 14 is located in the chuck 20 and the second tool seat 16 is located in the ram 40. In this embodiment, the first tool seat 14 has a sufficient extent in axial direction (sufficient depth) to concentrically support the driven end 55 of the tool even when the tool is retracted from the maximum insertion depth Dₘₐₓ, at which depth the driven portion 54 is fully inserted into the tool passage 12, to a retracted position at which position the tool retaining member 15 still engages the driven portion 54.

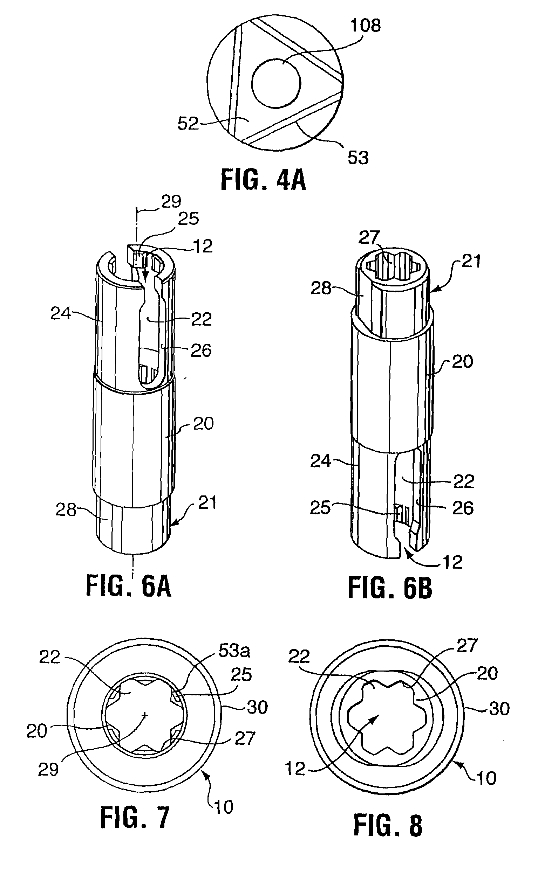

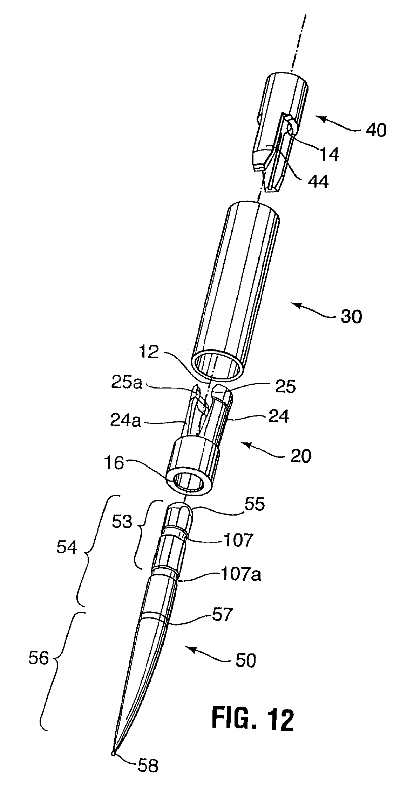

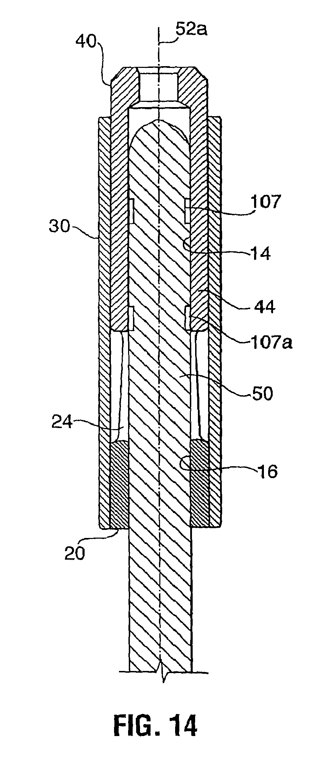

The tool retaining arm 24 is formed by a resilient portion of the chuck wall 13 surrounding the axial bore 22. The retaining arm 24 is preferably radially resiliently deflectable for insertion of the driven portion 56 into the bore 22. The retaining arm 24 preferably has a tool engaging tab 25 for contact with the contact surface 60 of the bur 50. The retaining arm is made of a sufficiently strong material (preferably stainless steel) to bias the tool engaging tab 25 against the contact surface 60 with sufficient force, once the driven portion 54 is inserted into the axial bore 22, to frictionally engage the bur 50 for torque transfer and to prevent axial movement of the bur 50 in the drive spindle 10 during operation of the handpiece 100. The selection of appropriate materials for the chuck 20 and the retaining arm 24 is not part of the present invention and is well within the abilities of the art skilled person. It will also be readily apparent to the art skilled person that the chuck 20 may be provided with multiple retaining arms 24, such as the pair of diametrically opposite retaining arms 24 shown in the embodiments of Figs. 6A *to 6D**.* In one embodiment, the retaining arm 24 extends towards the tool receiving end 23 of the chuck 20 in the spindle (see Figs. 10 and 11) and the tool engaging member 15 is located near the tool receiving end 23 for frictional engagement with the driven portion 56. This orientation allows for a larger retraction range than with a retaining arm 24 extending toward the driven chuck end 21 of the chuck 20 in the spindle (Figs. 12 and 14) since the bur 50 must still be held by the retaining arm 24 at maximum retraction.

In the embodiment of the tool drive arrangement of the invention shown in Figs. 10 and 11, the chuck 20 is constructed for interaction with the mechanical maximum retraction indicator 59 on the bur 50 to indicate Dₘᵢₙ. To that end, the tool engaging tab 25 protrudes radially inwardly from the retaining arm 24 and is sized and shaped to not only fit into the mechanical maximum extraction indicator 59, in this embodiment an indicator groove 70, but to also to generate a tactile sensation for the user to indicate that Dₘᵢₙ has been reached. In this manner, the user will preferably insert the bur 50 into the chuck 20 until Dₘₐₓ is reached, which is apparent from the fact that no further insertion of the bur is possible, and then retract the bur 50 to the desired position. Over-retraction of the bur 50 from the chuck 20 is avoided by the tactile sensation of the tool engaging tab 25 snapping into the indicator groove 70 which is felt, and in some cases heard, by the user when Dₘᵢₙ is reached.

Engagement of the tool engaging tab 25 of the retaining arm 24 with the indicator groove 70 also provides an additional safety feature not available in conventional handpiece designs. ISO recognizes excessive heat as one of the major contributing factors to chuck fatigue and failure in conventional handpieces. To avoid the generation of excessive heat by the user maintaining pressure on the bur release push button of a turbine handpiece, one of the ISO standards stipulates the minimum set back force of the push button resetting spring. The intention of that standard is to avoid friction between the push button mechanism and the spindle of the handpiece during rotation of the turbine. Excessive heat not only reduces lubrication, but more importantly can lead to relaxation of the spring force of the bur retaining arms of the chuck. Those arms are generally made of tempered steel and excessive heat leads to creep of the tempered material, thereby relaxing their resetting force. Once relaxation has occurred, the bur may no longer be reliably retained in the chuck. This problem is overcome with the embodiment of the tool drive arrangement of the invention shown in Figs. 10 and 11, for example, wherein the tool engaging tab 25 on the retaining arm 24 engages the indicator groove 70. This mechanical engagement can be achieved even after heat relaxation of the retaining arm 24, so that the bur 50 is more reliably retained in the chuck 20.

In one variant, as illustrated in Figs. 10 and 11, the bur 50 includes the indicator groove 70 as well as a maximum insertion groove 72 into which the tool engaging tab 25 snaps when the bur 50 is fully inserted into the chuck 20. This provides a tactile sensation to the user at both maximum insertion (Dₘₐₓ) and maximum retraction (Dₘᵢₙ) of the bur 50. In another variant (not illustrated), the bur 50, in addition to the indicator groove 70 and the maximum insertion groove 72, includes one or more intermediate insertion grooves located therebetween (not shown) which each cooperate with the tool engaging tab 25 to provide a tactile sensation to the user. These additional grooves can be spaced along the driven portion 54 at selected intervals to provide a 'depth indexing' or 'retraction length indexing' function. In yet a further variant (not illustrated), the multiple annular indexing grooves can be replaced with a helical indexing groove extending along the driven portion 56 of the bur 50, allowing for depth indexing of the bur by rotating the bur 50 relative to the chuck 20 while the tool engaging tab 25 is engaged in the helical groove. Multiple helical grooves can also be provided.

In the preferred embodiment exemplified in Figs. 9A and 9B, the chuck 20 includes a pair of diametrically opposed retaining arms 24 formed by two semi-circular wall portions of the chuck 20 which are separated by axial slits 26. The tool engaging tab 25 (see Figs. 6A and 6B) is formed by one or more protrusions extending radially inward from an inner surface of the retaining arms 24 about the central axis 29 for frictionally engaging the contact surface 60 of the driven portion 54 to prevent axial movement of the bur 50 during operation of the handpiece 100, and for axial engagement with the first axial end shoulder 68 of the maximum retraction indicator 59 (see Fig. 2) for indicating when Dₘᵢₙ is reached.

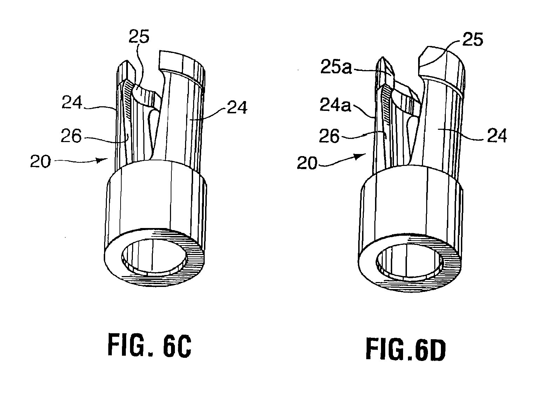

In another variant (Fig. 6C), the tool engaging tab 25 is formed by a pair of diametrically opposed annular ridges protruding from the inner surfaces of two opposed semi-circular retaining arms 24. The retaining arms and tool engaging tabs can be constructed and achieved by any means known to those skilled in the art. For instance, the retaining arms 24 may be straight with generally parallel axially extending sides, as shown in Figs. 6A and 6B, or they may be tapered as shown in Figs. 6C and 6D. It will further be appreciated by those of skill in the art that there may be more than two retaining arms, preferably arranged in a symmetrical fashion for vibration-free rotation at high speeds.

The tool engaging tabs 25 may be of any suitable shape and design, for example, they may be square or rectangular in profile (Figs. 10, 11), angled at one axial end in profile (Figs. 6A to 6C), angled at both axial ends in profile (Fig. 6D), or they may have a different shape altogether, such as rounded, so long as the retaining function is reliably achieved. To aid in guiding the bur 50 into the chuck 20, the surface of the tabs 25 at the tool receiving end of the retaining arms 24 can be angled toward the central axis 12 of the tool receiving bore 22, similar to the tab shown in Fig. 6D. In the tool drive arrangement illustrated in Fig. 12, which encompasses the chuck of Fig. 6D, the angled tab 25 actually aids in aligning the lugs 44 of the ram 40 within the axial slits 26 of the chuck 20 in the assembled condition of the spindle 10.

The depth to which a tool engaging tab 25 extends into a mechanical retraction indicator 59 or 59a, preferably a detent 51 or groove 70 on the bur 50, can be varied, for example, depending on design and materials. The tab height may be less than, equal to, or more than the depth of the groove. When the tab 25 height is equal to or less than the depth of the detent 51, or groove 70, this prevents excessive deformation of the retaining arms when the bur is inserted therebetween with the tab or tabs engaged in a mechanical indicator 59 or 59a. Such an arrangement also ensures maximal frictional engagement of the bur by the retaining arms for reduced rotational and axial slippage.

In the embodiment of the tool drive arrangement shown in Fig. 2, as seen in Figs. 9A and 9B, the drive spindle 10 further includes a ram 40 for radially forcing apart the retaining arms 24 during insertion and retraction or removal of the bur 50. In this embodiment, the ram 40 is mounted in the casing sleeve 30 at the tool insertion end 13 of the drive spindle 10 and is axially aligned with and adjacent to the chuck 20. The ram 40 has a central tool opening 42 for passage of the bur 50 and a pair of diametrically opposed lugs 44 extending axially from the ram 40 toward the chuck 20 in the assembled condition of the spindle 10. The lugs 44 are shaped to engage the axial slits 26 of the chuck 20 in the assembled condition. The lugs 44 are preferably longitudinally tapered to force apart the retaining arms 24 when the ram 40 and chuck 20 are forced toward one another. The ram 40 is preferably fastened to the casing sleeve 30 and the chuck 20 is preferably movable in the casing sleeve 30 to allow for movement of the chuck 20 relative to the ram 40 for use of the spindle 10 in pushbutton release handpieces. Activating the pushbutton of such a handpiece (not illustrated) will move the chuck 20 in the casing sleeve 30 toward the ram 40 whereby the retaining arms 24 are radially forced apart by the lugs 44, as will be readily apparent to the person skilled in the art. The ram 40 is permanently or releasably fastened to the casing sleeve 30, for example, by a threaded connection or a press-fit. Other possible fastening methods include welding or gluing and the like. However, the fastening method used must ensure that the ram 40 will not move relative to the casing sleeve 30 when the ram and chuck are forced against one another for the opening of the retaining arms 24.

In one variant of the spindle 10, the casing sleeve 30 represents the torque receiving element of the spindle. The sleeve 30 fits sufficiently closely into the spindle socket 109 of the drive head 101 (see Fig. 1) to ensure reliable torque transfer from the drive 105 to the spindle 10. Rotational torque is then transferred from the spindle 10 to the bur 50 through engagement of the lugs 44 of the ram 40 in the axial slits 26 of the chuck 20 and frictional engagement of the retaining arms 24 with the contact surface 60 on the bur 50. In a preferred variant of the spindle 10, as shown in Figs. 6A and 6B, torque is transferred directly from the drive 105 to the chuck 20 by way of a torque key 28 on the driven chuck end 21 of the chuck 20, which is shaped for fitting engagement with a torque socket (not illustrated) at a bottom of the spindle socket 109. The torque key 28 is formed by providing the driven chuck end 21 with any non-circular outer cross-section. The torque key 28 and the torque socket preferably have complementary shapes, but non-complementary shapes providing an interference fit can also be used as long as rotation of the torque key 28 relative to the torque socket is reliably prevented. In a preferred embodiment, the non-circular torque key 28 is shaped from a generally cylindrical end portion of the chuck 20 which is provided with two diametrically opposed flattened surface portions (see Figs. 6B and 8).

In a preferred embodiment of the drive arrangement, the drive arrangement further includes a structure for locking the bur 50 against rotation in the tool passage 12 of the drive spindle 10. This unique torque transfer arrangement is preferably combined with the torque key 28 and torque socket arrangement described directly above to provide for direct torque transfer from the drive 105 to the bur 50 without the possibility of any slippage and the associated heat generation and possible thermal damage to components of the drive arrangement, especially the tempered tool retaining arms 24. The torque transfer arrangement includes a locking portion 53 on the bur 50, which has an outer non-circular cross section (see Figs. 3D, 4A to 8, 10, 11) and a locking socket 27 in the chuck 20, which has a complementary or interlocking cross-section. The locking portion 53 is shaped to slidably fit into the locking socket 27 to allow length adjustment of the bur 50 by retracting the bur 50 from the fully inserted position in accordance with the principle aspect of the invention.

Preferably, the locking socket 27 is co-extensive with the first tool seat 14, which means the first tool seat 14 is shaped as a locking socket of a cross-sectional shape permitting fitting and slidable insertion of the locking portion 53 of the bur 50 while positively preventing rotation of the locking portion 53 in the locking socket 27. Fig. 4A shows an axial end view of a preferred embodiment of a dental bur 50 in accordance with the present invention having a locking portion 53 of triangular cross-section. Fig. 8 illustrates an end view of the drive spindle 10 of the preferred embodiment of Fig. 2, illustrating the cross-sectional shape of the locking socket 27 which is not directly complementary to the cross-section of the locking portion 53 shown in Fig. 4, but nevertheless provides an interference fit of the locking portion 53 in the locking socket 27 to guarantee a reliable interlocking between the locking portion 53 and the locking socket 27. In this embodiment, the locking socket 27 is a multi-faceted socket formed in a portion of the tool passage 12 of the chuck 20 near the driven end 21 of the chuck. The multi-faceted locking socket 27 provides multiple possible insertion orientations for the triangular lock portion 53 to improve the chance of aligning the locking portion 53 with the locking socket 27 without the aid of visual pre-alignment.

In a particularly preferred embodiment, the locking socket 27 extends substantially the whole length of the tool passage 12 for maintaining concentricity during rotation. It is preferable that the locking portion 53 and the locking socket 27 be rotation symmetrical, which means symmetrical about the axis of rotation to prevent excessive vibration of the bur 50 or chuck 20, and thus the handpiece, during high speed rotation. In the alternative, the locking portion 53 and/or the locking socket 27 can also be momentum symmetrical, which means weight balanced about the axis of rotation, again to prevent excessive vibration in the handpiece.

To improve the ease of proper alignment of the locking portion 53 with the locking socket 27, a particularly preferred embodiment of the chuck 20 includes a bur aligning member 53 a (Fig. 7) near the bur insertion end of the drive spindle 10. The bur aligning member 53a preferably corresponds in shape and orientation with the non-circular locking socket 27, which is generally located deep in the drive head of the handpiece. This bur aligning member 53a allows for pre-alignment of the locking portion 53 with the locking socket 27 upon insertion of the driven portion 54 into the drive spindle 10. The bur aligning member 53a forms part of the tool engaging tab 25 in the embodiment shown in Fig. 6.

In an alternate preferred embodiment of the tool drive arrangement of the invention, shown in Fig. 12, all parts perform the same function, although the chuck 20 and ram 40 are positioned in the spindle 10 in an axially opposite orientation to that in the embodiment of Fig. 2. The bur 50 is inserted first into the chuck 20 and subsequently enters the axially aligned and adjacent ram 40. In this orientation, the first tool seat 14 is formed in the ram 40 and a portion of the sleeve 30 for supporting the driven end 55 of the tool during length adjustment, and the second tool seat 16 is located in the chuck 20 for supporting the driven portion 54 of the tool at a position between the driven end 55 and the working portion 56.

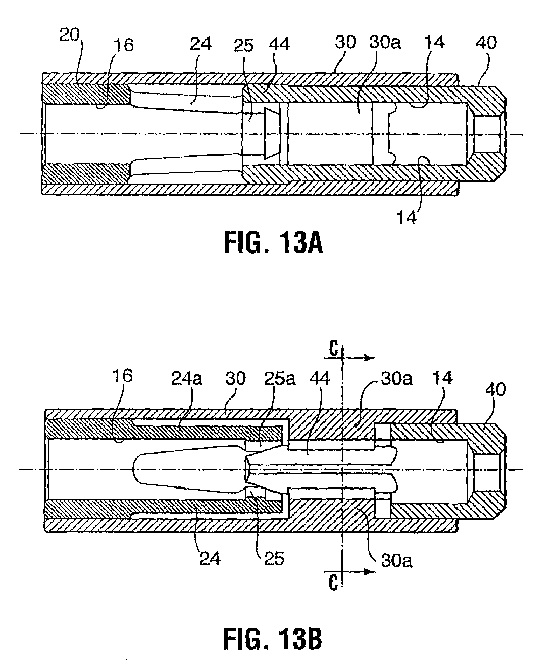

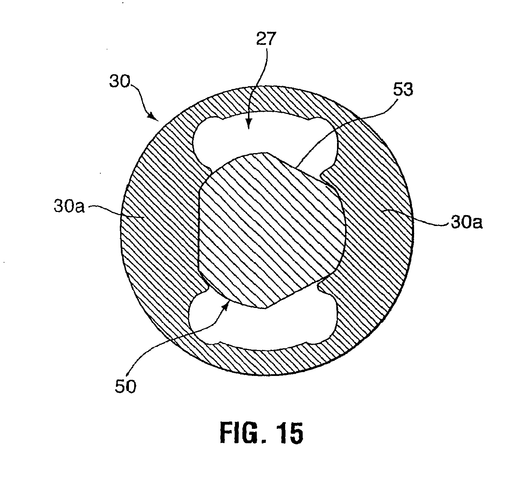

In the variant illustrated in Fig. 12, drive torque is transferred to the sleeve 30 through frictional engagement with the spindle socket 109, for example by press-fitting the spindle 10 into the spindle socket 109. The ram 40 is securely fitted into the spindle and engages the chuck 20 in an orientation wherein lugs 44 extending from the ram 40 toward the chuck 20 engage axial slits 26 formed in the chuck 20, similar to the embodiment shown in Fig. 2. However, torque is transferred differently from the embodiment of Fig. 2. For torque transfer in the embodiment of Fig 12, as shown in Figs. 13B and 15, a constricted portion 30a of the casing sleeve 30 provides a locking socket 27 to prevent rotation of the locking portion 53 bur 50 relative to the sleeve 30. The locking socket 27 can be designed in any suitable manner. For instance, as shown in Fig. 15, the constricted portion 30a can have a non-circular cross-section complementary to the non-circular cross-section of the locking portion 53 of the bur 50 or, alternatively, it can provide an interference fit to form a locking socket 27, as described elsewhere above. The constricted portion 30a also prevents rotation of the ram 40, specifically the lugs 44, relative to the sleeve 30. This not only maintains the ram in the same rotational position in the sleeve 30 at all times, but also the chuck 20 due to the interaction between the lugs 44 of the ram 40 and the axial slits 26 in the chuck 20.

In the tool drive arrangement of Fig. 12, as shown in Fig. 13B, a tool engaging tab 25 projects from one retaining arm 24, while the second retaining arm 24a has a relatively flattened tab 25a, which essentially acts as a pressure pad against the contact surface 60 of the mechanical indicator 59 of the bur 50 during operation. The pressure pad may be in the form of a flattened tab 25a, as shown, or it may simply be a retaining arm without any tab. A single tab 25 plus pressure pad 25a arrangement, which means an asymmetrical tab arrangement, is preferred for burs 50 which have the mechanical retraction indicator 59 located on the locking portion 53 of the bur 50 (see Fig 3G). In this embodiment, the depth of the detent 51 is asymmetrical about the circumference of the locking portion 53 of the bur 50 due to the non-circular cross-section of the locking portion 53 (see Figs. 4B and 4C). During operation, the bur 50 is oriented in the spindle 10 such that the tab 25 engages the deeper portion of the detent 51 and the pressure pad 25a engages the shallow portion of the detent 51 where the surface of the bur 50 has been flattened to form the triangular locking portion 53 (Fig. 4C). This orientation is achieved by the specific shape of the locking socket 27 in the sleeve 30 as shown in Fig. 15. The provision of asymmetrical tabs 25 and 25a, as in this embodiment, is especially advantageous for tools with three-sided non-circular locking portions. The use of asymmetrical tabs and a shaped locking socket 27 which forces the bur 50 into the same rotational position relative to the chuck 20 significantly reduces wear. Use of a symmetrical chuck having identical tabs would result in one tab always being in contact with a flattened locking surface on the tool while the other would engage the circular external surface of the tool shaft in the locking portion 56, resulting in wear on that external surface.

A person skilled in the art will appreciate that an asymmetric or single-tab chuck must be counterbalanced to prevent excessive vibration during rotation, in particular at the high rotation speeds encountered with an air turbine handpiece. This can be achieved by balancing the weight of the retaining arms 24 and 24a, or preferably, by balancing the overall spindle system about the central axis for smooth rotation. For example, material can be removed, added, or repositioned in one or more of the sleeve 30, the chuck 20 or the ram 40, to accommodate for any difference in weight between the two retaining arms 24 and 24a, or to balance any other asymmetrical components of the spindle 10. In the embodiment shown in Figs. 13B and 15, the sleeve has been designed to counterbalance the system due to the asymmetrical design of the chuck. The design of the locking socket 27 is also counterbalanced to prevent vibration during rotation.

In an alternative embodiment to Fig. 12 (not shown), the ram is in torque-receiving communication with the drive mechanism in the handpiece by way of a torque key 28, similar to the torque key 28 on the chuck 20 of the embodiment shown in Fig. 2. The locking socket 27 in this alternate preferred embodiment (not shown) is preferably located within the tool-receiving bore of the ram 40 but may also be located in the sleeve 30, similar to the constricted portion 30a of the sleeve 30 shown in Fig. 13B. The locking socket 27 is preferably elongated and radially supports the bur 50 to maintain concentricity during rotation at various insertion depths between Dₘᵢₙ and Dₘₐₓ. The socket is preferably complementary in shape to the non-circular cross-sectional locking portion 53 of the bur 50, or provides an interference fit similar to that exemplified in Figs. 6A and 6B.

As shown in Fig. 15, the constricted portion 30 of the sleeve 30 forming the locking socket for torque transfer to the bur 50 has an asymmetrical shape to always align the bur in the socket in the same orientation relative to the sleeve. In particular, the cross-sectional shape of the constricted portion 30a includes a flat portion for engagement with a flattended section on the locking portion 53 of the bur 50, which flat portion is diametrically opposite a circular portion of sufficient diameter to fittingly engage a externally circular section of the locking portion 53. The spacing of the diametrically opposite flat and circular portions of the locking socket in the sleeve 30 (constricted portion 30a) is selected to be substantially equal to the dimensions of the locking portion 53 of the bur 50 so that the locking portion is fittingly insertable into the locking socket and locked against rotation therein for reliable torque transfer from the sleeve 30 to the bur 50.

Other non-circular cross-sectional locking portions and complementary locking sockets are also contemplated, for example, square-, rectangle-, octagonal-, diamond-, star-, and flattened circle-shape among others. A non-circular locking portion can also have a generally circular shape with one or more indents, notches or axial grooves projecting radially inward into the locking portion 53. A variant in which the locking portion 53 of the bur 50 directly engages a locking socket 27 formed in a portion of the drive mechanism, for example a turbine, for direct torque transfer is also contemplated.

It is contemplated that a dental tool in accordance with the present invention can have any type of working tip for contacting a tooth surface known in the art. Furthermore, a portion or all of the tool may be provided with a wear resistant coating. One or more of the components of the rotatable tool drive arrangement of the present invention may be provided with a low friction coating, for example the lugs 44 of the ram 40. It is contemplated that a tool according to the present invention may further comprise an axial channel to allow passage of air or liquid from the handpiece to a surface of a tooth. It is also contemplated that the tool of the invention may be a tool other than a dental bur.

The invention relates to an improved dental tool drive arrangement for a handpiece with a drive head, the tool drive arrangement permitting length adjustment of the tool in the drive head by concentrically supporting the tool in the drive head at any position from a fully inserted position to a maximum retracted position. The tool drive arrangement preferably includes a tool and a rotatable tool supporting element for concentrically supporting the tool from the fully inserted to the maximum retracted position, the tool preferably including a maximum retraction indicator for indicating to a user when the tool has been retracted to the maximum retraction position. This provides a significant advantage over the prior art by allowing a user to adjust the exposed length of a rotatable tool, preferably a dental bur, without exceeding safe operating limits. The invention also relates to an improved drive spindle which allows depth adjustment of a tool in a dental handpiece while maintaining efficient torque transfer and concentricity during high speed rotation.

### The invention is further described by the following embodiments:

Embodiment 1. A rotatable tool drive assembly for use with a dental or medical handpiece having a drive head with an axis of rotation, the assembly comprising
   a rotatable tool; and
   a tool drive arrangement for releasably supporting the tool, the tool drive arrangement being insertable into the drive head for coaxial rotation in the drive head; the tool having a tool body with an axis of rotation, a driven portion with a driven end for insertion into the tool supporting element and a working portion for projecting from the drive head during use; the tool drive arrangement having a tool passage for coaxially receiving the driven portion of the tool at different insertion depths, the tool passage including a first tool seat for concentrically supporting the driven end of the tool and a second tool seat for concentrically supporting the driven portion at a location intermediate the driven end and the working portion; the first tool seat being axially elongated for concentrically supporting the driven end at any position from a maximum insertion depth wherein the tool is fully inserted into the tool passage to a maximum retraction depth wherein the tool is retracted from the maximum insertion depth.
Embodiment 2. The tool drive assembly with the features of embodiment 1, wherein the tool includes a maximum retraction indicator for indicating to a user when the tool has been retracted to the maximum retraction depth.
Embodiment 3. The rotatable tool drive assembly with the features of embodiment 2, wherein the tool drive arrangement includes a drive spindle for being fittingly received in a drive spindle socket of the drive head, the drive spindle having a drive torque receiving portion for receiving drive torque from the drive head, a tool supporting portion connected with the drive torque receiving portion and having the tool passage with first and second tool seats, the drive spindle further including a tool retaining member for releasably retaining the driven portion in the tool passage; and the axial length of the first tool seat being selected for the tool retaining member to engage the driven portion at any position of the tool from the maximum insertion position to the maximum retraction position.
Embodiment 4. A rotatable dental tool for use in a tool drive assembly in accordance with claim 1, the tool comprising
   a tool body having an axis of rotation, the tool body divided into a driven portion with a driven end for insertion into the tool drive arrangement and a working portion with a working end for projection from the drive head during use; and a maximum retraction indicator on the driven portion for indicating to a user when the tool is retracted from the supporting portion to the maximum retraction depth.
Embodiment 5. The tool with the features of embodiment 4, wherein the maximum retraction indicator is a visible indicia located on the driven portion, the maximum retraction indicator being located intermediate the driven end and the working portion to be hidden from view when the tool is inserted at a depth between the maximum insertion depth and maximum retraction depth and visible to a user when the tool is inserted into the handpiece to a depth equal to the maximum retraction depth or less.
Embodiment 6. The tool with the features of embodiment 5, wherein the maximum retraction indicator is selected from the group consisting of a dot, a line, a colored line, an etched line, a laser mark, a line having a surface roughness different from the remainder of the driven portion, a detent and a groove.
Embodiment 7. The tool with the features of embodiment 4, wherein the maximum retraction indicator is a mechanical indicia located on the driven portion for engagement by a portion of the tool supporting element when the maximum retraction depth is reached.
Embodiment 8. The tool with the features of embodiment 7, wherein the mechanical indicia is a stop on the driven portion for mechanical interaction with the supporting element when the tool is retracted to the maximum retraction depth.
Embodiment 9. The tool with the features of embodiment 8, further comprising
   a contact surface on the driven portion for engagement by the tool engaging member at insertion depths at least from the maximum retraction depth to the maximum insertion depth;
   the stop being a stop shoulder on the contact surface for axial engagement by the tool engaging member when the tool is retracted from the maximum insertion depth to the maximum retraction depth.
Embodiment 10. The tool with the features of embodiment 9, wherein the contact surface has an axial extent for engagement by the tool engaging member at any tool insertion depth from the maximum retraction depth to the maximum insertion depth.
Embodiment 11. The tool with the features of embodiment 8, wherein the maximum retraction indicator is a detent on the driven portion and the stop is a stop shoulder that projects radially outward from the contact surface.
Embodiment 12. The tool with the features of embodiment 11, wherein the stop shoulder projects radially outward from the contact surface at an obtuse angle to the axis of rotation.
Embodiment 13. The tool with the features of embodiment 11, wherein the detent is an annular groove and the stop shoulder is a lateral wall of the groove.
Embodiment 14. The tool with the features of embodiment 8, wherein the stop shoulder is an annular projection extending radially outward from the contact surface.
Embodiment 15. The tool with the features of embodiment 8, wherein the driven portion includes a torque lock portion having a non-circular, rotation symmetrical cross-section for engagement in a torque socket of complementary shape in the drive spindle.
Embodiment 16. The tool with the features of embodiment 15, wherein the torque lock portion has a non-circular cross-section.
Embodiment 17. The tool with the features of embodiment 15, wherein the torque lock portion has a polygonal cross-section.
Embodiment 18. The tool with the features of embodiment 16, wherein the non-circular cross-section of the torque lock portion is formed by at least two circumferentially evenly distributed non-cylindrical surface portions.
Embodiment 19. The tool with the features of embodiment 18, wherein the non-circular surface portions are selected from the group of flat surfaces, notches, grooves, indents, ridges and bars.
Embodiment 20. The tool with the features of embodiment 17, wherein the torque lock portion forms a major part of the driven portion.
Embodiment 21. The tool with the features of embodiment 4, for use in an air turbine driven handpiece.
Embodiment 22. The tool with the features of embodiment 21, wherein the tool is a dental bur.
Embodiment 23. A tool for use in a dental handpiece having a rotatable drive and a drive spindle for receiving the tool and a retaining member for frictionally engaging the tool in the spindle, the tool comprising
   a tool body having an axis of rotation, a working portion for projecting from the handpiece and a driven portion for insertion into the drive spindle to a maximum insertion depth,
   a detent on the working portion for engagement by the retaining member,
   the detent having a first axial end shoulder, the shoulder being located on the driven portion for indicating a pre-selected maximum retraction depth of the driven portion which is less than the maximum insertion depth.
Embodiment 24. A drive spindle for use in a tool drive assembly according to claim 1, the drive spindle comprising
   a torque receiving element for receiving rotational torque from the drive;
   a tool supporting element connected with the torque receiving element and having a tool passage for receiving the driven portion of the tool coaxial with an axis of rotation of the spindle; and a tool retaining member connected to the tool supporting element for releasably engaging the driven portion to releasably retain the tool in the supporting element;
   the tool passage including a first tool seat for supporting the driven end of the tool and a second tool seat for supporting the driven portion at a location intermediate the driven end and the working portion; the first tool seat having an axial length for concentrically supporting the driven end when the tool is retracted from a maximum insertion depth at which the driven portion is fully inserted into the tool passage to a retracted position at which the tool retaining member still engages the driven portion.
Embodiment 25. The drive spindle with the features of embodiment 24, wherein the axial length of the first tool seat is at least 10% of the axial length of the driven portion, preferably 15-60%, most preferably 20-70%.
Embodiment 26. The drive spindle with the features of embodiment 24, wherein the axial length of the first tool seat is at least 1.5mm, preferably at least 2mm, more preferably 2-7mm, most preferably about 5mm.
Embodiment 27. A drive spindle for rotatably supporting a tool in a dental handpiece having a rotational torque generating drive, the tool having a body divided into a driven portion with a driven end for insertion into the handpiece and a working portion for extending from the handpiece during use, the drive spindle comprising
   a driving element for receiving drive torque from the torque generating drive;
   a tool supporting element connected with the driving element and having a tool passage for receiving the driven portion of the tool coaxial with an axis of rotation of the spindle, the tool passage including a first tool seat for supporting the driven end of the tool and a second tool seat for supporting the driven portion at a location intermediate the driven end and the working portion; the first tool seat having an axial length for concentrically supporting the driven end when the tool is retracted from a maximum insertion depth at which the driven portion is fully inserted into the tool passage to a reduced insertion depth at which the tool retaining member still engages the driven portion; and
   a tool engaging member for releasably engaging the driven portion to releasably retain the tool in the tool passage between an engagement depth at which depth the contact between the tool engaging member and the driven portion is initiated and the maximum insertion depth; the tool engaging member being positioned in the spindle for engagement of the driven portion of the tool at a location closer to the working portion than the driven end.
Embodiment 28. The drive spindle with the features of embodiment 27, wherein the driving element is a chuck for receiving the tool and having means for transferring drive torque to the tool.
Embodiment 29. The drive spindle with the features of embodiment 28, wherein the chuck is also the tool supporting element.
Embodiment 30. The drive spindle with the features of embodiment 27, wherein the driving element is a ram and the tool supporting element is an axially aligned chuck, the ram engaging the chuck for rotational torque transfer.
Embodiment 31. The drive spindle with the features of embodiment 27, wherein the driving element is a generally cylindrical sleeve for receiving the driven portion of the tool and the supporting element is a chuck coaxial with the sleeve.
Embodiment 32. The drive spindle with the features of embodiment 27, wherein the tool engaging member is located on an inner surface of a wall portion of the chuck, the wall portion being expandable upon insertion of the driven portion, such that insertion of the driven portion into the bore forces the wall portion to expand radially outward, the expanded wall portion applying force radially inward against the driven portion such that the tool engaging member frictionally engages the driven portion for torque transfer and to prevent axial movement of the driven portion relative to the chuck.
Embodiment 33. The drive spindle with the features of embodiment 27, wherein the tool engaging member comprises one or more protrusions extending radially inward from the inner surface of the wall portion, the tool engaging member either continuously or discontinuously extending annularly about the axis of rotation.
Embodiment 34. The drive spindle with the features of embodiment 32, wherein the wall portion further comprises a pair of diametrically opposed axial retaining arms and wherein a portion of the tool engaging member is located on an inner surface of one or both of the retaining arms extending into the bore.
Embodiment 35. The drive spindle with the features of embodiment 34, wherein the retaining arms are formed by two semi-circular wall portions of the chuck separated by axial slits.
Embodiment 36. The drive spindle with the features of embodiment 34, wherein the tool engaging member is a tab or a discontinuous annularly extending ridge.
Embodiment 37. The drive spindle with the features of embodiment 28 or 29, further comprising a ram axially aligned with and adjacent to the chuck in the drive spindle, the ram operatively engaging the chuck in a torque transfer arrangement.
Embodiment 38. The drive spindle with the features of embodiment 37, wherein the torque transfer arrangement consists of a pair of diametrically opposed lugs for engaging the axial slits in the adjacent wall portion of the chuck.
Embodiment 39. The drive spindle with the features of embodiment 31 for use with a tool having a non-circular section on the driven portion, wherein the cylindrical sleeve has a central bore and a pair of torque transfer projections projecting radially into the bore for torque transferring engagement with the non-circular section on the tool.
Embodiment 40. The drive spindle with the features of embodiment 31, wherein the ram is fitted into the sleeve for drive torque transfer from the sleeve to the ram.
Embodiment 41. The drive spindle with the features of embodiment 28, wherein the chuck is in direct contact with the drive for torque transfer to rotate the tool in the handpiece.
Embodiment 42. The drive spindle with the features of embodiment 27, wherein the driven portion is in direct contact with the drive for torque transfer to rotate the tool in the handpiece.
Embodiment 43. The drive spindle with the features of embodiment 30, wherein the ram is in direct contact with the drive for torque transfer to rotate the tool in the handpiece.
Embodiment 44. The drive spindle with the features of embodiment 27, further comprising a locking socket of non-circular cross-section for engaging a complementary non-circular cross-sectional portion of the driven portion to lock the tool against rotation in the drive spindle.
Embodiment 45. The drive spindle with the features of embodiment 44, wherein the locking socket is a multi-faceted socket for providing a plurality of engagement orientations with a triangular cross-sectional portion of the driven portion.
Embodiment 46. The drive spindle with the features of embodiment 28, wherein the means for transferring drive torque is a locking socket of non-circular cross-section for engaging a complementary non-circular cross-sectional portion of the driven portion on the tool, which locking socket is located in a portion of the tool receiving bore of the chuck.
Embodiment 47. The drive spindle with the features of embodiment 45, wherein the locking socket is elongated in the axial tool receiving bore such that the lock portion of the tool engages the locking socket at any insertion depth between the maximum retraction depth and the maximum insertion depth.
Embodiment 48. The drive spindle with the features of embodiment 30, further comprising a locking socket of non-circular cross-section for engaging a complementary non-circular cross-sectional portion of the driven portion to lock the tool against rotation in the drive spindle, the locking socket being located in a portion of the ram.
Embodiment 49. The drive spindle with the features of embodiment 44, further comprising a tool aligning member positioned in the tool receiving bore near a tool insertion end of the drive spindle for aiding in pre-alignment of the locking portion with the locking socket, the tool aligning member being complementary in shape and orientation with the locking socket.
Embodiment 50. The drive spindle with the features of embodiment 24 or 27, wherein the tool is a dental bur.
Embodiment 51. A dental handpiece having a handle for gripping by a user, the handle in communication with a drive head, the drive head housing a drive for providing torque to rotate a dental tool in the handpiece, the handpiece comprising the drive spindle as defined in claim 24 or 27.
Embodiment 52. A dental bur for use in a dental handpiece having a rotatable drive and a drive spindle for receiving the bur and a retaining member for frictionally engaging the bur in the spindle, the bur comprising a tool body having an axis of rotation, the tool body divided into a driven portion with a driven end for insertion into the tool supporting element and a working portion with a working end for projection from the drive head during use; and a maximum retraction indicator on the driven portion for indicating to a user when the tool is retracted from the supporting portion to the maximum retraction depth.

The above-described embodiments of the present invention are intended to be examples only. Alterations, modifications and variations may be effected to the particular embodiments by those of skill in the art without departing from the scope of the invention, which is defined solely by the claims appended hereto.

## Claims

1. A rotatable bur drive assembly for use with a dental or medical handpiece having a drive head with an axis of rotation, the assembly comprising
a rotatable bur; and
a bur drive arrangement for releasably supporting the bur, the bur drive arrangement being insertable into the drive head for coaxial rotation in the drive head; the bur having a tool body with an axis of rotation, a driven portion with a driven end for insertion into the bur drive arrangement and a working portion with a working end for projecting from the drive head during use; the bur drive arrangement having a chuck for releasably supporting the bur, a resilient tool retaining member and a ram for selectively releasing the bur from the chuck, the chuck having a tool passage for coaxially receiving the driven portion of the bur at different insertion depths, the tool passage including a first bur seat for concentrically supporting the driven end of the bur and a second bur seat being located in the ram for concentrically supporting the driven portion of the bur at a location intermediate the driven end and the working portion; the chuck having a tool receiving end and the tool retaining member extending towards the tool receiving end; the first bur seat being axially elongated for concentrically supporting the driven end at any position from a maximum insertion depth wherein the drive end is fully inserted into the tool passage to a maximum retraction depth wherein the bur is retracted from the maximum insertion depth and the drive end is still engaged in the first tool seat; and the axial length of the first bur seat being selected for the bur retaining member to engage the driven portion at any position of the bur from the maximum insertion position to the maximum retraction position.

2. The bur drive assembly of claim 1, wherein the bur includes a maximum retraction indicator for indicating to a user when the bur has been retracted to the maximum retraction depth.

3. The rotatable bur drive assembly of claim 2, wherein the bur drive arrangement includes a drive spindle for being fittingly received in a drive spindle socket of the drive head, the drive spindle having a drive torque receiving portion for receiving drive torque from the drive head, a bur supporting portion connected with the drive torque receiving portion and having the first and second bur seats, and the tool retaining member.

4. A rotatable dental bur for use in a bur drive arrangement having a tool passage for coaxially receiving the bur and including first and second bur seats for concentrically supporting the bur, the first bur seat being axially elongated for concentrically supporting the bur at different insertion depths, the bur comprising
a tool body having an axis of rotation, the tool body divided into a driven portion with a driven end for insertion into the tool passage of the bur drive arrangement and a working portion with a working end for projection from the bur drive arrangement during use; the driven portion including a torque lock portion having a non-circular, rotation symmetrical or momentum symmetrical cross-section for engagement in a torque socket of the bur drive arrangement providing an interference fit; and a maximum retraction indicator on the driven portion for indicating to a user when the bur is retracted from the supporting portion to a maximum retraction depth wherein the drive end is only partially inserted in the first bur seat.

5. The bur of claim 4, wherein the maximum retraction indicator is a visible indicia located on the driven portion, the maximum retraction indicator being located intermediate the driven end and the working portion to be hidden from view when the bur is inserted at a depth between the maximum insertion depth and maximum retraction depth and visible to a user when the bur is inserted into the handpiece to a depth equal to the maximum retraction depth or less.

6. The bur of claim 5, wherein the maximum retraction indicator is selected from the group consisting of a dot, a line, a colored line, an etched line, a laser mark, a line having a surface roughness different from the remainder of the driven portion, a detent and a groove.

7. The bur of claim 4, wherein the maximum retraction indicator is a mechanical indicator located on the driven portion for engagement by a portion of the bur supporting element when the maximum retraction depth is reached.

8. The bur of claim 7, wherein the mechanical indicator is a stop on the driven portion for mechanical interaction with the supporting element when the bur is retracted to the maximum retraction depth.

9. The bur of claim 8, further comprising
a contact surface on the driven portion for engagement by the bur engaging member at insertion depths at least from the maximum retraction depth to the maximum insertion depth;
the stop being a stop shoulder on the contact surface for axial engagement by the tool engaging member when the bur is retracted from the maximum insertion depth to the maximum retraction depth.

10. The bur of claim 9, wherein the contact surface has an axial extent for engagement by the bur engaging member at any bur insertion depth from the maximum retraction depth to the maximum insertion depth.

11. The bur of claim 8, wherein the maximum retraction indicator is a detent on the driven portion and the stop is a stop shoulder that projects radially outward from the contact surface.

12. The bur of claim 11, wherein the stop shoulder projects radially outward from the contact surface at an obtuse angle to the axis of rotation.

13. The bur of claim 11, wherein the detent is an annular groove and the stop shoulder is a lateral wall of the groove.

14. The bur of claim 8, wherein the stop shoulder is an annular projection extending radially outward from the contact surface.

15. The tool of claim 8, wherein the mechanical indicator is located on the locking portion of the bur.

16. The bur of claim 4, wherein the torque lock portion has a non-circular cross-section.

17. The bur of claim 4, wherein the torque lock portion has a polygonal cross-section.

18. The bur of claim 4, wherein the non-circular cross-section of the torque lock portion is formed by at least two circumferentially evenly distributed non-cylindrical surface portions.

19. The bur of claim 18, wherein the non-circular surface portions are selected from the group of flat surfaces, notches, grooves, indents, ridges and bars.

20. The bur of claim 17, wherein the torque lock portion forms a major part of the driven portion.

21. The bur of claim 4, for use in an air turbine driven handpiece.

22. The bur of claim 21, wherein the bur is a dental bur.

23. A bur for use in a dental handpiece having a rotatable drive and a drive spindle for receiving the tool and a retaining member for frictionally engaging the bur in the spindle and a torque lock socket for providing an interference fit with the bur, the bur comprising
a tool body having an axis of rotation, a working portion for projecting from the handpiece and a driven portion for insertion into the drive spindle to a maximum insertion depth,
the driven portion including a torque lock portion having a non-circular, rotation symmetrical or momentum symmetrical cross-section for engagement in the torque socket of the spindle providing an interference fit; and
a detent on the driven portion for engagement by the retaining member,
the detent having a first axial end shoulder, the shoulder being located on the driven portion for indicating a pre-selected maximum retraction depth of the driven portion which is less than the maximum insertion depth.

24. A dental handpiece, comprising a handle for gripping by a user, the handle in communication with a drive head, the drive head housing a bur drive arrangement for releasably supporting a bur having a tool body with an axis of rotation, a driven portion with a driven end for insertion into the bur drive arrangement and a working portion with a working end for projecting from the drive head during use; the bur drive arrangement having a chuck for releasably supporting the bur, a resilient tool retaining member and a ram for selectively releasing the bur from the chuck, the chuck having a tool passage for coaxially receiving the driven portion of the bur at different insertion depths, the tool passage including a first bur seat for concentrically supporting the driven end of the bur and a second bur seat being located in the ram for concentrically supporting the driven portion of the bur at a location intermediate the driven end and the working portion; the chuck having a tool receiving end and the tool retaining member extending towards the tool receiving end; the first bur seat being axially elongated for concentrically supporting the driven end at any position from a maximum insertion depth wherein the drive end is fully inserted into the tool passage to a maximum retraction depth wherein the bur is retracted from the maximum insertion depth and the drive end is still engaged in the first tool seat; and the axial length of the first bur seat being selected for the bur retaining member to engage the driven portion at any position of the bur from the maximum insertion position to the maximum retraction position.
